# EUROPEAN PATENT SPECIFICATION

(11) **EP 1 646 404 B1**
(45) Date of publication and mention of the grant of the patent: **13.01.2010**
(21) Application number: 04749149.3
(22) Date of filing: 08.07.2004
(51) Int. Cl.: A61K 47/36, A61K 47/34, A61K 9/08, A61K 9/14

(54) **SOLID COMPOSITION COMPRISING A PROTON PUMP INHIBITOR**
FESTE ZUSAMMENSETZUNG MIT EINEM PROTONENPUMPENHEMMER
COMPOSITION SOLIDE COMPRENANT UN INHIBITEUR DE LA POMPE A PROTONS

(30) Priority: 11.07.2003 US 486795 P
(43) Date of publication of application: 19.04.2006
(73) Proprietor: AstraZeneca AB, 151 85 Södertälje (SE)
(72) Inventor: BLYCHERT, Eva, 431 83 Mölndal (SE); JANSSEN, Marjo, 2719 EE Zoetermeer (NL)
(86) International application number: PCT/SE2004/001113
(87) International publication number: WO 2005/004921

(56) References cited:
- WO-A1-00/06122
- WO-A1-88/06893
- WO-A1-94/25070
- WO-A1-98/24414
- WO-A1-03/009846
- WO-A2-95/34292
- US-A- 5 817 338

## Description

### Field of the invention.

The present invention relates to the use of a composition comprising an acid labile heterocyclic compound with gastric acid inhibitory effect, in the following referred to as a proton pump inhibitor compound, and a pharmaceutical composition for administration via a gastric tube comprising such a compound. The composition and use are useful in the treatment of gastrointestinal disorders and aimed especially for treatment of patients with difficulties to swallow and for pediatric patients.

### Background of the invention and prior art.

Proton pump inhibitor compounds having effect as H⁺K⁺-ATPase inhibitors are for instance compounds known under the generic names omeprazole, lansoprazole, pantoprazole, rabeprazole, tenatoprazole and esomeprazole.

These active substances are useful for inhibiting gastric acid secretion in mammals and man. In a more general sense, they may be used for prevention and treatment of gastric acid related diseases in mammals and man, including e.g. reflux esophagitis, gastritis, duodenitis, gastric ulcer and duodenal ulcer. Furthermore, they may be used for treatment of other gastrointestinal disorders where gastric acid inhibitory effect is desirable e.g. in patients on NSAID therapy, in patients with Non Ulcer Dyspepsia, in patients with symptomatic gastro-esophageal reflux disease, and in patients with gastrinomas. They may also be used in patients in intensive care situations, in patients with acute upper gastrointestinal bleeding, pre-and postoperatively to prevent acid aspiration of gastric acid and to prevent and treat stress ulceration. Further, they may be useful for prevention and treatment of irritable bowel syndrome (IBS), inflammatory bowel disease (IBD), ulcerative colitis, Crohn's disease, asthma, laryngitis, Barret's syndrome, sleep apnea, sleep disturbance, psoriasis as well as being useful for prevention and treatment of Helicobacter infections and diseases related to the above.

These active compounds are, however, susceptible to degradation/transformation in acidic and neutral media. The degradation is catalyzed by acidic compounds and is stabilized in mixtures with alkaline compounds. The stability of the active substances is also affected by moisture, heat, organic solvents and to some degree by light.

With respect to the stability properties of the active substances, it is obvious that an oral dosage form should be protected from contact with the acidic gastric juice or comprise suitable components to neutralise the acidic gastric juice so that the active substance can be transferred in intact form to that part of the gastrointestinal tract where pH is near neutral and where rapid absorption can occur.

A pharmaceutical oral dosage form of such an acid labile H⁺K⁺-ATPase inhibitor is best protected from contact with acidic gastric juice by an enteric coating layer. For oral administration, commonly used solid dosage forms are capsules and tablets comprising a multitude of enteric coated pellets of the active ingredient. For instance the following US patents, US 4,853,230, US 4,786,505, US 5,817,338 and US 5,753,265 describe suitable enteric coated preparations. Said preparations contain a core comprising the active ingredient or an alkaline salt thereof, optionally together with an alkaline reacting material, this core is layered with a separating layer and an enteric coating layer. The separating layer may be an optional feature. In order to further enhance the storage stability the prepared formulation may optionally be packed with a desiccant.

However, tablets and capsules are less suitable for administration to patients with difficulties to swallow and for pediatric use. Several of the proton pump inhibitors can be administered orally after dispersion in an aqueous liquid, such as water, fruit juice and fruit sauce. Some of the marked proton pump inhibitors such as omeprazole and lansoprazole, are approved for administration via nasogastric tube, but there is still a need for improvement. For administration via a naso-gastric tube, the content of a Prevacid ® capsule, i.e. the enteric coated pellets of lansoprazole, will be emptied into 40 mL apple juice and injected through the tube. However, only tubes with a relatively large inner diameter, e.g. CH16 (CH= Cherrier), are suitable for this type of administration.

The tableted dosage form described in US 5,817,338, which is marked under the trade name Losec® MUPS® and Prilosec^{™} OTC, is also suitable for administration via naso-gastric tube after being dispersed in water to a suspension, whereas the prescribed product marked in USA under the trade name Prilosec^{®} , a capsule comprising enteric coated pellets of omeprazole can only be administered through a tube after the content of the capsule has been dispersed in a buffering solution. Further, the product sold under the trade name Prevacid^{®} Oral-suspension should not be given through tube according to the instruction from the manufacture.

Furthermore, there is recommendation for instance in WO 04/004718 to administer micro-granules from Lansoprazole^{®} fast dissolving tablet through a naso-gastric tube after being suspended in 10 mL distilled water or apple juice and flushing the tube with additionally 10 mL of the liquid.

Problems that might arise with administration of enteric coated pellets through gastric tube are for instance caused by the size of the enteric coating layered pellets and the inner diameter the tube or the outlet of the syringe, which might cause clogging in the syringe or tube. This is especially critical for pediatric patients where thin tubes are often required. There is also a risk of reduced patient compliance and non-complete dose delivery because of pellets sediment in the glass and/or clogging the syringe used when preparing the suspension. This is especially critical in pediatric use when working with small volumes and doses.

In most cases, parental and /or injectable formulations are not viable alternative because of the need for administration to patients by people trained in medical care and in hospitals.

Therefore, it is still a demand for an improved method for oral administration and for development of new enteric coating layered multiple unit dosage forms that can be used for oral administration. The dosage forms intended for the new administration route should fulfil high demand with respect to chemical and mechanical stability during an extended storage time. Furthermore, there is still a demand for dosage forms having improved patient acceptance, which are easy to handle. One demand on solid dosage forms and compositions is that they should be dispersible in liquids making them possible for oral administration to young children and patients with difficulties to swallow. Thus, it is essential to have a robust method, which will enable patients in an easy way to prepare a suspension and to have a liquid vehicle that is easy provided and which will not interfere with the medicament in a negative way.

### Brief description of the invention.

One object of the present invention is to provide use of a composition comprising enteric coating layered pellets of a proton pump inhibition for administration via a gastric tube aimed for pediatric use. The expression gastric tube includes naso-gastric tubes as well as other tubes or syringes aimed for feeding a suspension or dispersion into the stomach of a patient.

According to one feature of the invention, the enteric coated pellets of the proton pump inhibitor are mixed with a viscous medium. The proton pump inhibitor compound is selected from the group of compounds omeprazole, lansoprazole, pantoprazole, rabeprazole, tenatoprazole or esomeprazole. According to a further feature the compound is esomeprazole prepared for oral administration, preferably as esomeprazole magnesium trihydrate in the form of enteric coating layered pellets. According to another feature the enteric coated pellets are admixed with a solid pharmaceutical acceptable thickener and the thickener is capable of forming a viscous medium when dispersed in an aqueous carrier.

Another object of the present invention is to provide a solid composition, which comprises a proton pump inhibitor compound in the form of a multiple of enteric coating layered pellets in admixture with one or more thickeners capable of forming a viscous medium when dispersed in an aqueous barrier. Prior to administration, a ready-to-use composition is prepared by mixing an aqueous carrier with the solid composition consisting of the pharmaceutically active proton pump inhibitor in admixture with one or more thickeners.

The viscosity of the medium or the formed aqueous medium comprising the dispersed or suspended enteric coating layered pellets of the active substance has an impact on the feeding of the suspension through a tube, such as a gastric or naso-gastric tube. Surprisingly, it has been found that the higher viscosity the thinner tubes can be used within certain limits. The enteric coated layered pellets of the active substance are usually in the form of spherical pellets which, in a liquid medium, tend to accumulate very tightly to each other due to their uniform form. This 'piling up' can potentially clog the tube. The viscous dispersing medium will enable the enteric coated pellets to float within the medium. It has been found that an enhanced 'floating' property will enable the pellets to be passed through thinner tubes. A solid composition comprising a thickener, which forms a viscous medium upon mixing with an aqueous carrier, facilitates and improves the administration, especially to young children. For instance, the handling instruction to patients can be simpler and the administration can be less time consuming. This will reduce the occurrence of clogging of the enteric coated pellets in the tubes.

### Detailed description of the invention.

### Active substance:

Compounds of interest for the present invention are H⁺K⁺ ATPase inhibitor compounds of the follow formulas or an alkaline salt thereof or one of its single enantiomers or an alkaline salt or the single enantiomer thereof.

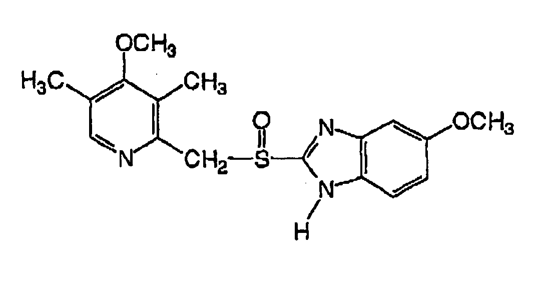

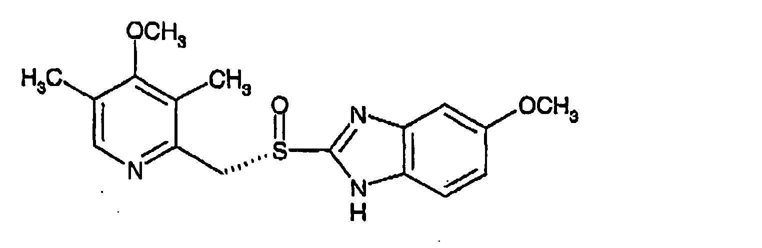

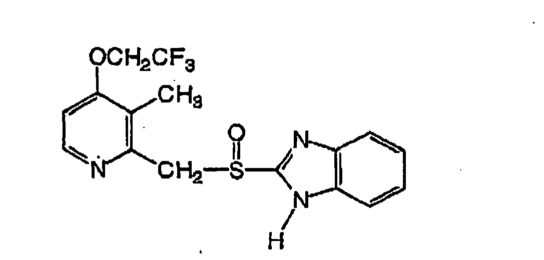

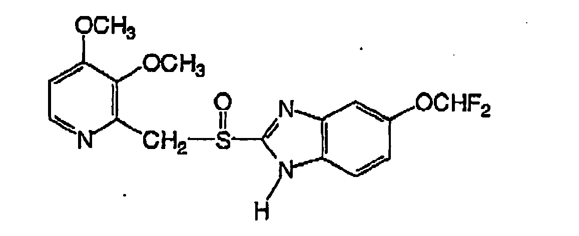

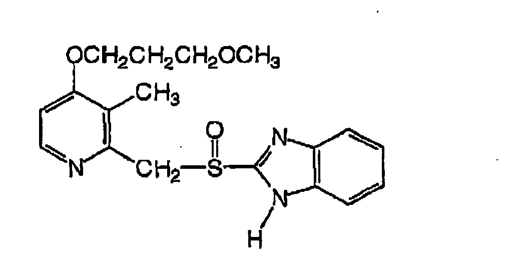

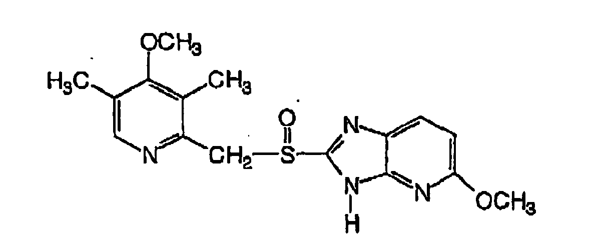

The active compound used in the claimed composition may be used in neutral form or in the form of an alkaline salt, such as for instance the Mg²⁺, Ca²⁺, Na⁺ or K⁺ salts, preferably the Mg²⁺ salts. The compounds may also be used in the form of one of its single enantiomers or alkaline salts thereof, such as exemplified in the second structural formula above.

Thus, the proton pump inhibitors for the claimed invention are selected from the group of omeprazole, lansoprazole, pantoprazole, rabeprazole, tenatoprazole or a pharmaceutical acceptable salt thereof or a single enantiomer thereof, such as esomeprazole magnesium.

Some of the above mentioned compounds are for instance disclosed in EP-A1-0005129, EP-A1-174726, EP-A1-166287, GB 2163747, WO 94/27988, WO95/01977 and WO98/54171.

### Enteric coating layered pellets:

### Core material

The core material for the individually enteric coating layered pellets can be constituted according to different principles. Seeds layered with active substance, optionally mixed with alkaline compounds, can be used as the core material for the further processing.

The seeds, which are to be layered with the active substance, can be water insoluble seeds comprising different oxides, celluloses, organic polymers and other materials, alone or in mixtures or water soluble seeds comprising different inorganic salts, sugars, non-pareils and other materials, alone or in mixtures. The seeds are usually spherical spheres. Further, the seeds may comprise active substance in the form of crystals, agglomerates, compacts etc. According to one aspect of the invention the size of the seeds, e.g. the spherical spheres, should be less than approximately 0.8 mm, and according to a further aspect the seeds are less than 0.4 mm. The seeds layered with active substance are produced either by powder- or solution/suspension layering using for instance granulating or spray coating/layering equipment.

Before the seeds are layered, the active substance may be mixed with further components. Such components can be binders, surfactants, fillers, disintegrating agents, alkaline additives or other pharmaceutically acceptable ingredients, alone or in mixtures. The benders are for example celluloses such as hydroxypropyl methylcellulose, hydroxypropyl cellulose and carboxymethyl-cellulose sodium, polyvinyl pyrrolidone, sugars, starches and other pharmaceutically acceptable substances with cohesive properties. Suitable surfactants are found in the groups of pharmaceutically acceptable non-ionic or ionic surfactants such as for instance sodium lauryl sulfate.

Alternatively, the proton pump inhibitor or one of its single enantiomers or an alkaline salt thereof, optionally mixed with alkaline compounds and further mixed with suitable constituents can be formulated into core material. Said core materials may be produced by extrusion/spheronization, balling or compression utilizing different equipments. According to one aspect of the invention the size of the formulated core materials is approximately less than 1 mm, according to a further aspect in the range between 0.5 - 1 mm or less than 0.5 mm. The manufactured core materials can further be layered with additional ingredients comprising active substance and/or be used for further processing.

The active substance is mixed with pharmaceutical constituents to obtain preferred handling and processing properties and a suitable concentration of active substance in the final mixture. Pharmaceutical constituents such as fillers, binders, lubricants, disintegrating agents, surfactants and other pharmaceutically acceptable additives, can be used.

The active substance may also be mixed with an alkaline pharmaceutically acceptable substance (or substances). Such substances can be chosen among, but are not restricted to, substances such as the sodium, potassium, calcium, magnesium and aluminium salts of phosphoric acid, carbonic acid, citric acid or other suitable weak inorganic or organic acids; aluminium hydroxide/sodium bicarbonate coprecipitate; substances normally used in antacid preparations such as aluminium, calcium and magnesium hydroxides; magnesium oxide or composite substances, such as Al₂O₃.6MgO.CO₂.12H₂O, (Mg₆Al₂(OH)₁₆CO₃.4H₂O), MgO.Al₂O₃. 2SiO₂.nH₂O or similar compounds; organic pH-buffering substances such as trihydroxymethylaminomethane, basic amino acids and their salts or other similar, pharmaceutically acceptable pH-buffering substances.

Alternatively, the aforementioned core material can be prepared by using spray drying or spray congealing technique.

### Enteric coating layer(s)

Before applying enteric coating layer(s) onto the core material in the form of individual pellets, said pellets may optionally be covered with one or more separating layers comprising pharmaceutical excipients optionally including alkaline compounds such as for instance pH-buffering compounds. This/these separating layer(s) separate(s) the core material from the outer layer(s) being enteric coating layer(s).

The separating layer(s) can be applied to the core material by coating or layering procedures in suitable equipments such as coating pan, coating granulator or in a fluidized bed apparatus using water and/or organic solvents for the coating process. As an alternative the separating layer(s) can be applied to the core material by using powder coating technique. The materials for separating layers are pharmaceutically acceptable compounds such as, for instance, sugar, polyethylene glycol, polyvinylpyrrolidone, polyvinyl alcohol, polyvinyl acetate, hydroxypropyl cellulose, methyl-cellulose, ethylcellulose, hydroxypropyl methyl cellulose, carboxymethylcellulose sodium and others, used alone or in mixtures. Additives such as plasticizers, colorants, pigments, fillers, anti-tacking and anti-static agents, such as for instance magnesium stearate, titanium dioxide, talc and other additives may also be included into the separating layer(s).

When the optional separating layer(s) is applied to the core material it may constitute a variable thickness. The maximum thickness of the optional separating layer(s) is normally only limited by processing conditions. The separating layer(s) may serve as a diffusion barrier and may act as a pH-buffering zone. The pH-buffering properties of the separating layer(s) can be further strengthened by introducing into the layer(s) substances chosen from a group of compounds usually used in antacid formulations such as, for instance, magnesium oxide, hydroxide or carbonate, aluminium or calcium hydroxide, carbonate or silicate; composite aluminium/magnesium compounds such as, for instance Al₂O_{3.}6MgO.CO_{2.}12H₂O, (Mg₆Al₂(OH)₁₆CO₃.4H₂O), MgO.Al₂O_{3.}2SiO₂.nH₂O, aluminium hydroxide/sodium bicarbonate coprecipitate or similar compounds; or other pharmaceutically acceptable pH-buffering compounds such as, for instance the sodium, potassium, calcium, magnesium and aluminium salts of phosphoric, carbonic, citric or other suitable, weak, inorganic or organic acids; or suitable organic bases, including basic amino acids and salts thereof. Talc or other compounds may be added to increase the thickness of the layer(s) and thereby strengthen the diffusion barrier. The optionally applied separating layer(s) is not essential for the invention. However the separating layer(s) may improve the chemical stability of the active substance and/or the physical properties of the novel multiple unit tableted dosage form.

One or more enteric coating layers are applied onto the core material or onto the core material covered with separating layer(s) by using a suitable coating technique. The enteric coating layer material may be dispersed or dissolved in either water or in suitable organic solvents. As enteric coating layer polymers one or more, separately or in combination, of the following can be used; e.g., solutions or dispersions of methacrylic acid copolymers, cellulose acetate phthalate, hydroxypropyl methylcellulose phthalate, hydroxypropyl methylcellulose acetate succinate, polyvinyl acetate phthalate, cellulose acetate trimellitate, carboxymethylethylcellulose, shellac or other suitable enteric coating layer polymer(s).

The enteric coating layers contain pharmaceutically acceptable plasticizers to obtain the desired mechanical properties, such as flexibility and hardness of the enteric coating layers. Such plasticizers are for instance, but not restricted to, triacetin, citric acid esters, phthalic acid esters, dibutyl sebacate, cetyl alcohol, polyethylene glycols, polysorbates or other plasticizers.

The amount of plasticizer is optimized for each enteric coating layer formula, in relation to selected enteric coating layer polymer(s), selected plasticizer(s) and the applied amount of said polymer(s), in such a way that the mechanical properties, i.e. flexibility and hardness of the enteric coating layer(s) are adjusted. Additives such as dispersants, colorants, pigments, polymers e.g. poly(ethylacrylat, methylmethacrylat), anti-tacking and antifoaming agents may also be included into the enteric coating layer(s). Other compounds may be added to increase film thickness and to decrease diffusion of acidic gastric juices into the acidic susceptible material.

To protect an acidic susceptible substance, such as a proton pump inhibitor and to obtain an acceptable acid resistance of the multiple unit dosage form according to the invention, the enteric coating layer(s) constitutes a thickness of approximately at least 10 µm, preferably more than 20 µm. The maximum thickness of the applied enteric coating layer(s) is normally only limited by processing conditions.

### Over-coating layer

Pellets covered with enteric coating layer(s) may further be covered with one or more over-coating layer(s). The over-coating layer(s) can be applied to the enteric coating layered pellets by coating or layering procedures in suitable equipments such as coating pan, coating granulator or in a fluidized bed apparatus using water and/or organic solvents for the layering process. The materials for over-coating layers are pharmaceutically acceptable compounds such as, for instance sugar, polyethylene glycol, polyvinylpyrrolidone, polyvinyl alcohol, polyvinyl acetate, hydxoxypropyl cellulose, methylcellulose, ethylcellulose, hydroxypropyl methylcellulose, carboxymethylcellulose sodium and others, used alone or in mixtures. Additives such as plasticizers, colorants, pigments, fillers, anti-tacking and anti-static agents, such as for instance magnesium stearate, titanium dioxide, talc and other additives may also be included into the over-coating layer(s). Said over-coating layer may further prevent potential agglomeration of the enteric coating layered pellets and protect the enteric coating layer from incompatible excipients or pH values above its dissolution pH, e.g. during the preparation of the suspension, administration and swallowing. The over-coating layer may also include a taste-masking agent. The maximum thickness of the optionally applied over-coating layer(s) is normally only limited by processing conditions.

### Pharmaceutically acceptable thickener:

Thickeners suitable for the composition of the present invention are thickeners generally used in food industry, such as starch, carrageenan, xanthan gum, guar gum, locust bean gum, tragacanth, gelatin, pectin, and modified cellulose derivatives which form a viscous medium upon mixing with an aqueous carrier. One single thickener or a combination of thickeners can be used.

Alternatively, the enteric coated pellets comprising the pharmaceutically active ingredient may be directly mixed with a vehicle, which is easy to provide for and which will not interfere with the active ingredient and/or the enteric coated pellets. Such a suitable vehicle is for instance a viscous aqueous medium such as yoghurt, syrup, sour milk or any aqueous liquid with a similar viscosity. According to one aspect of the invention the vehicle is sugar syrup with a specified amount of sugar to provide for a suitable viscosity. The viscous medium formed or used will provide a homogenous suspension/dispersion to allow the enteric coated pellets floating in the medium.

The pH value of the viscous medium should be adjusted so that the enteric coating of the pellets is not destroyed during the administration and/or preparation of the aqueous suspension or dispersion of pellets in the viscous medium, i.e. the enteric coating polymer should not be dissolved in the viscous medium. This is specifically important if ther is no protective over-coating layer applied on the enteric coating layered pellets. According to one aspect of the invention, the pH value of the viscous medium is approximately less than 7 and according to a further aspect less than pH 5.6.

The viscous medium or the thickener as such may be mixed with suitable flavour and colour agents and/or sweetening agents acceptable for food products.

It has surprisingly been found that the higher viscosity of the aqueous medium being formed or used for the dispersion/suspension of the enteric coating pellets the narrower gastric tubes can be used.

According to one feature of the invention, the commercial available thickener sold under the trade name "Thick-It" comprising maize starch has shown to be suitable for the improved method for administration via gastric tube. According to another feature, the viscous medium could be an aqueous vehicle, such as yoghurt, syrup or sour milk. Such a suitable aqueous vehicle could be sugar syrup with a sugar content of at least 63% by weight to provide for a suitable viscosity of the medium.

According to one aspect of the invention, the viscosity of the formulation after gelation should be 0.005 - 10 Pa s and preferably 0.05 - 5 Pa s, as determined at a shear rate of 10 s⁻¹ from a flow-curve recorded on a rheometer equipped with a plate-plate geometry. Alternatively, the viscosity can be expressed as amount of thickener with respect to amount of aqueous liquid.

Suitable aqueous carriers or liquids to be used for mixing with the thickener and for administration of the composition through a gastric tube are water, and other pharmaceutically acceptable carriers for oral administration such as fruit juices, dairy products such as milk. Alternatively, the aqueous carrier as such can be used as viscous medium. Suitable vehicles are sour milk, yoghurt, syrup and liquids with similar viscosity.

The amount of administered aqueous carrier/liquid depends on the amount of active substance, but will generally be in the range of 1 - 50 mL, preferably 1 - 30mL.

### Gastric tubes:

Gastric tubes includes naso-gastric tubes as well as tubes for feeding. Tubes suitable for the improved method of administration are tubes made of polyvinyl cellulose, polyurethan and similar materials. The size of the tube can vary depending on the patients and the purpose.

Suitable size for pediatric use is approximately a size of CH5 - CH10, such as CH5, CH6 and CH8.

### Use of composition.

The composition according to the invention is used for reducing gastric acid secretion. It can be administered one to several times a day. The typical daily dose of the active substance varies and will depend on various factors such as the individual requirements of the patients, the mode of administration and disease. In general the daily dose will be in the range of 1-100 mg of active substance, in some severe cases such as Zollinger-Ellison syndrome there might be a need for higher doses. Preferred doses for adults are 10 - 80 mg and for pediatric use the preferred doses are 0.5 - 40 mg of active substance, and for the youngest 0.5 - 20 mg depending on the severity.

The present invention is further described and exemplifies in the following experimental report without limiting the invention. The scope of protection is defined by the accompanying claims.

### Experimental report.

### Equipment:

Luer lock ™ syring: 30mL.
Tube: Pennine™ health care, Ref No. 15E00; 020E01 (CH 8); 13B01 (CH 10) Flocare™ Pure tube, Ref No. 35242 (CH 6) Argyle Salem Sump ™ (CH 10)
Graduated glass: 100mL
Thickener: Thick-It™
Tap water for mixing with thickener.
Sugar syrup: 630 g Sucrose, 1 g Methylis Parahydroxbenzoas, 369 g Water.
Yoghurt

### Preparation of enteric coated pellets:

Esomeprazole magnesium trihydrate was prepared according to US 6,369,085. Omeprazole magnesium was prepared accorodng to US 5,900,424. The prepared active ingredients were formulated into enteric coated pellets, respectively as described in US 5,817,338, and the prepared pellets were mixed with the prepared viscous media.

### Preparation of viscous medium comprising Thick-It^{™} ;

Different amounts of Thick-It ™ (5, 6, 7 and 8 g, respectively) were mixed with 100 mL tap water, the aqueous solution was mixed vigorously for approx. 1 minute. Prepared enteric coated pellets comprising esomeprazole Mg, corresponding to 10 mg esomeprazole were mixed with 10mL of the different aqueous, viscous media prepared. The different suspensions were feeded through different tubes.

Pure tap water without thickener was used as reference medium.

### The tubes were tested according to the following procedure:

The tubes were flushed with some water before administration.
1. Remove the piston from a 25-50 mL syringe (Luer-Lock, 30 mL is used) and fill the syringe with approximately 25 mL of the prepared aqueous medium.
2. Empty the content of the capsule (esomeprazole enteric coated pellets) in the syringe and put the piston back, Leave a space of approximately 5 mL air.
3. Immediately shake the syringe for approximately 15 seconds to disperse the pellets.
4. Hold the syringe with the tip up and check that the tip has not been clogged.
5. Attach the syringe to the tube whilst maintaining the above position (tip pointing up).
6. Shake the syringe and position it with the tip pointing down. Immediately inject 5-10 mL of the mixture into the tube. Invert the syringe after injection and shake (the syringe must be held with the tip pointing up to avoid clogging of the tip). Tum the syringe with the tip down and immediately inject another 5-10 mL of the mixture into the tube. Repeat this procedure until the syringe is empty.
7. Fill the syringe with 25 mL of water and 5 mL of air and repeat step 6 if necessary to wash down any sediment left in the syringe.

### Result:

When comparing results from the testing of aqueous media with and without thickener, the enteric coated pellets could be administered through more narrow tubes without clogging the tubes when using viscous media for feeding than when using pure tap water. In this experiment, optimal viscosity was obtained when using 6 - 7 g Thick-It ™ in 100 mL tap water.

### Test with Sugar syrup and Yoghurt:

Adminstration of enteric coated pellets comprising omeprazole magnesium were tested according to the following:
The tubes were flushed with some water before administration. The tablet/granules were added in the syringe, then the liquid (sugar syrup or yoghurt) was pulled into the syringe. In the case of a tablet, the tablet was left for 2 min. to disintegrate. This enables optimal hygiene and minimal utensils. The mixture could be passed down the tube in one big push preferably without pauses, the pauses may introduce stacking of the pellets.

Multiple unit tablets or capsules comprising enteric coated pellets of omeprazole magnesium were mixed alternatively with sugar syrup or yoghurt and were tested with good results in gastric tubes of a size down to CH 8. Before administration, the pellets were mixed with 15 mL sugar syrup and 5 mL yoghurt, respectively.

## Claims

1. Use of a composition comprising an acid labile proton pump inhibitor compound in the form of a multiple of enteric coating layered pellets in the preparation of a medicament in the form of an aqueous suspension for the treatment of gastrointestinal disorders, wherein the proton pump inhibitor compound is selected from the group of compounds known under the generic names omeprazole, lansoprazole, pantoprazole, rabeprazole, tenatoprazole and esomeprazole or a pharmaceutically acceptable salt thereof and the pellets are in admixture with one or more pharmaceutically acceptable thickeners selected from the group of starch, xanthan gum, carrageenan, guar gum, locust bean gum, tragacanth, gelatin, pectin and modified cellulose derivatives dispersed in an aqueous carrier for administration through a gastric tube to pediatric patients in need of such a treatment.

2. The use according to claim 1, wherein the thickener is selected from starch and xanthan gum.

3. The use according to claim 1, wherein the composition in addition comprises pharmaceutically acceptable additives selected from flavouring agent, colour agent and sweetening agent.

4. Use of a composition comprising an acid labile proton pump inhibitor compound in the form of a multiple of enteric coating layered pellets in the preparation of a medicament for treatment of gastrointestinal disorders, wherein the proton pump inhibitor compound is selected from the group of compounds known under the generic names omeprazole, lansoprazole, pantoprazole, rabeprazole, tenatoprazole and esomeprazole or a pharmaceutically acceptable salt thereof the pellets are dispersed in a pharmaceutically acceptable viscous aqueous medium selected from yoghurt, sour milk, syrup and aqueous liquids with a similar viscosity for administration through a gastric tube to pediatric patients in the need of such treatment.

5. The use according to claim 4, wherein the viscous aqueous medium is a sugar syrup with a sugar content of at least 63% by weight.

6. The use according to any of claims 1 and 4, wherein the viscosity of the formulation after gelation should be 0.005 - 10 Pa s, as determined at a shear rate of 10 s⁻¹ from a flow-curve recorded on a rheometer equipped with a plate-plate geometry.

7. The use according to any of claims 1 and 4, wherein the viscosity of the formulation after gelation should be 0.05 - 5 Pa s, as determined at a shear rate of 10 s⁻¹ from a flow-curve recorded on a rheometer equipped with a plate-plate geometry.

8. The use according to any of claims 1 and 4, wherein the aqueous suspension is administered through tubes with the size CH 5 to CH 10 (CH= Cherrier).

9. The use according to any of claims 1 - 8, wherein the amount of administered dose of the active substance is 0.5 - 40 mg.

10. The use according to claim 1, wherein the aqueous carrier is selected from the group of water, fruit juice, syrup and dairy products.

11. The use according to any of claims 1 -8, wherein the amount of administered viscous medium is approximately 1 - 35 mL.

12. A pharmaceutical formulation for administration through a gastric tube to pediatric patients comprising a proton pump inhibitor in the form of enteric coating layered pellets, wherein the proton pump inhibitor compound is selected from the group of compounds known under the generic names omeprazole, lansoprazole, pantoprazole, rabeprazole, tenatoprazole and esomeprazole or a pharmaceutically acceptable salt thereof and the pellets are in admixture with one or more pharmaceutically acceptable thickeners selected from the group of starch, xanthan gum, carrageenan, guar gum, locust bean gum, tragacanth, gelatin, pectin and modified cellulose derivatives dispersed in an aqueous carrier.

13. The use according to any of claims 1 - 8, wherein the enteric coated pellets are spherical and have a size of less than 1 mm.

14. The use according to any of claims 1 - 8, wherein the enteric coated pellets are spherical and have a size of less than 0.5 mm.

15. Use of a thickener in the preparation of a viscous aqueous composition for administration via a gastric tube for the treatment of gastrointestinal disorders, wherein the composition comprises a proton pump inhibitor is selected from the group of compounds known under the generic names omeprazole, lansoprazole, pantoprazole, rabeprazole, tenatoprazole and esomeprazole or a pharmaceutically acceptable salt thereof and it is in the form of a multiple of enteric coating layered pellets, in mixture with one or more thickeners selected from the group of starch, xanthan gum, carrageenan, guar gum, locust bean gum, tragacanth, gelatin, pectin and modified cellulose derivatives, and an aqueous carrier.

16. Use of a viscous medium in the preparation of a viscous aqueous composition for administration via a gastric tube for the treatment of gastrointestinal disorders, wherein the composition comprises a proton pump inhibitor is selected from the group of compounds known under the generic names omeprazole, lansoprazole, pantoprazole, rabeprazole, tenatoprazole and esomeprazole or a pharmaceutically acceptable salt thereof and it is in the form of a multiple of enteric coating layered pellets in mixture with a viscous aqueous medium selected from yoghurt, sour milk, syrup and aqueous liquids with a similar viscosity.

## Patentansprüche

1. Verwendung einer Zusammensetzung, die einen säurelabilen Protonenpumpenhemmer in Form von mehreren magensaftresistent beschichteten Pellets in der Zubereitung eines Medikaments in Form einer wäßrigen Suspension zur Behandlung von Erkrankungen des Magen-Darm-Trakts enthält, wobei der Protonenpumpenhemmer ausgewählt ist aus der Gruppe von Verbindungen, die unter den generischen Namen Omeprazol, Lansoprazol, Pantoprazol, Rabeprazol, Tenatoprazol und Esomeprazol bekannt sind, sowie pharmazeutisch annehmbaren Salzen davon, und die Pellets als eine Mischung mit einem oder mehreren pharmazeutisch annehmbaren Verdickungsmitteln ausgewählt aus der aus Stärke, Xanthangummi, Carrageenan, Guargummi, Johannisbrotgummi, Traganth, Gelatine, Pektin und modifizierten Zellulosederivaten bestehenden Gruppe dispergiert in einem wäßrigen Träger vorliegen, für die Verabreichung mittels einer Magensonde an pädiatrische Patienten, die einer solchen Behandlung bedürfen.

2. Verwendung nach Anspruch 1, wobei das Verdickungsmittel aus Stärke und Xanthangummi ausgewählt ist.

3. Verwendung nach Anspruch 1, wobei die Zusammensetzung zusätzlich pharmazeutisch annehmbare Zusatzstoffe ausgewählt aus Geschmacksstoffen, Farbstoffen und Süßstoffen enthält.

4. Verwendung einer Zusammensetzung, die einen säurelabilen Protonenpumpenhemmer in Form von mehreren magensaftresistenten beschichteten Pellets in der Zubereitung eines Medikaments zur Behandlung von Erkrankungen des Magen-Darm-Trakts enthält, wobei der Protonenpumpenhemmer ausgewählt ist aus der Gruppe von Verbindungen, die unter den generischen Namen Omeprazol, Lansoprazol, Pantoprazol, Rabeprazol, Tenatoprazol und Esomeprazol bekannt sind, sowie pharmazeutisch annehmbaren Salzen davon, wobei die Pellets in einem pharmazeutisch annehmbaren zähflüssigen wäßrigen Medium ausgewählt aus Joghurt, Sauermilch, Sirup und wäßrigen Flüssigkeiten mit einer ähnlichen Viskosität, dispergiert sind, für die Verabreichung mittels einer Magensonde an pädiatrische Patienten, die einer solchen Behandlung bedürfen.

5. Verwendung nach Anspruch 4, wobei es sich bei dem zähflüssigen wäßrigen Medium um einen Zuckersirup mit einem Zuckergehalt von wenigstens 63 Gew.-% handelt.

6. Verwendung nach Anspruch 1 oder 4, wobei die Viskosität der Formulierung nach dem Gelieren 0,005-10 Pa s betragen sollte, bestimmt bei einer Schergeschwindigkeit von 10 s⁻¹ aus einer Fließkurve aufgezeichnet mit einem Rheometer mit Platte-Platte-Geometrie.

7. Verwendung nach Anspruch 1 oder 4, wobei die Viskosität der Formulierung nach dem Gelieren 0,05-5 Pa s betragen sollte, bestimmt bei einer Schergeschwindigkeit von 10 s⁻¹ aus einer Fließkurve aufgezeichnet mit einem Rheometer mit Platte-Platte-Geometrie.

8. Verwendung nach Anspruch 1 oder 4, wobei die wäßrige Suspension über Schläuche mit der Größe CH 5 bis CH 10 (CH = Cherrier) verabreicht wird.

9. Verwendung nach einem der Ansprüche 1-8, wobei die Menge an verabreichter Wirkstoffdosis 0,5 - 40 mg beträgt.

10. Verwendung nach Anspruch 1, wobei der wäßrige Träger aus der aus Wasser, Fruchtsaft, Sirup und Milchprodukten bestehenden Gruppe ausgewählt ist.

11. Verwendung nach einem der Ansprüche 1-8, wobei die Menge an verabreichtem zähflüssigem Medium ungefähr 1 - 35 ml beträgt.

12. Pharmazeutische Formulierung zur Verabreichung über eine Magensonde an pädiatrische Patienten, umfassend einen Protonenpumpenhemmer in Form von magensaftresistent beschichteten Pellets, wobei der Protonenpumpenhemmer ausgewählt ist aus der Gruppe von Verbindungen, die unter den generischen Namen Omeprazol, Lansoprazol, Pantoprazol, Rabeprazol, Tenatoprazol und Esomeprazol bekannt sind, sowie pharmazeutisch annehmbaren Salzen davon, und die Pellets in einer Mischung mit einem oder mehreren pharmazeutisch annehmbaren Verdickungsmitteln ausgewählt aus der aus Stärke, Xanthangummi, Carrageenan, Guargummi, Johannisbrotgummi, Traganth, Gelatine, Pektin und modifizierten Zellulosederivaten bestehenden Gruppe, dispergiert in einem wäßrigen Träger vorliegen.

13. Verwendung nach einem der Ansprüche 1-8, wobei die magensaftresistent beschichteten Pellets kugelförmig sind und eine Größe von weniger als 1 mm aufweisen.

14. Verwendung nach einem der Ansprüche 1-8, wobei die magensaftresistent beschichteten Pellets kugelförmig sind und eine Größe von weniger als 0,5 mm aufweisen.

15. Verwendung eines Verdickungsmittels bei der Herstellung einer zähflüssigen wäßrigen Zusammensetzung für die Verabreichung mittels einer Magensonde zur Behandlung von Erkrankungen des Magen-Darm-Trakts, wobei die Zusammensetzung einen Protonenpumpenhemmer ausgewählt aus der Gruppe von Verbindungen, die unter den generischen Namen Omeprazol, Lansoprazol, Pantoprazol, Rabeprazol, Tenatoprazol und Esomeprazol bekannt sind, sowie pharmazeutisch annehmbaren Salzen davon, enthält, und in Form von mehreren magensaftresistent beschichteten Pellets als eine Mischung mit einem oder mehreren Verdickungsmitteln ausgewählt aus der aus Stärke, Xanthangummi, Carrageenan, Guargummi, Johannisbrotgummi, Traganth, Gelatine, Pektin und modifizierten Zellulosederivaten bestehenden Gruppe, und einem wäßrigen Träger vorliegt.

16. Verwendung eines zähflüssigen Mediums bei der Herstellung einer zähflüssigen wäßrigen Zusammensetzung zur Verabreichung mittels einer Magensonde zur Behandlung von Erkrankungen des Magen-Darm-Trakts, wobei die Zusammensetzung einen Protonenpumpenhemmer ausgewählt aus der Gruppe von Verbindungen, die unter den generischen Namen Omeprazol, Lansoprazol, Pantoprazol, Rabeprazol, Tenatoprazol und Esomeprazol bekannt sind, sowie pharmazeutisch annehmbaren Salzen davon, enthält, und in Form von mehreren magensaftresistent beschichteten Pellets als eine Mischung mit einem zähflüssigen wäßrigen Medium ausgewählt aus Joghurt, Sauermilch, Sirup und wäßrigen Flüssigkeiten mit einer ähnlichen Viskosität vorliegt.

## Revendications

1. Utilisation d'une composition comprenant un composé inhibiteur de la pompe à protons labile aux acides sous la forme d'un multiple de pastilles revêtues d'enrobage entérique dans la préparation d'un médicament sous la forme d'une suspension aqueuse destinée au traitement de troubles gastro-intestinaux, **caractérisée en ce que** le composé inhibiteur de la pompe à protons est choisi dans le groupe des composés connus sous les noms génériques oméprazole, lansoprazole, pantoprazole, rabéprazole, ténatoprazole et ésoméprazole ou un sel pharmaceutiquement acceptable de ceux-ci et les pastilles sont en mélange avec un ou plusieurs épaississants pharmaceutiquement acceptables choisis dans le groupe comprenant l'amidon, la gomme xanthane, le carraghénane, la gomme guar, la gomme de caroube, la tragacanthe, la gélatine, la pectine et les dérivés de cellulose modifiée dispersés dans un support aqueux pour l'administration par un tube gastrique à des patients pédiatriques ayant besoin d'un tel traitement.

2. Utilisation selon la revendication 1,
**caractérisée en ce que** l'épaississant est choisi parmi l'amidon et la gomme xanthane.

3. Utilisation selon la revendication 1,
**caractérisée en ce que** la composition comprend en outre des additifs pharmaceutiquement acceptables choisis parmi un agent aromatisant, un agent colorant et un agent édulcorant.

4. Utilisation d'une composition comprenant un composé inhibiteur de la pompe à protons labile aux acides sous la forme d'un multiple de pastilles revêtues d'enrobage entérique dans la préparation d'un médicament destiné au traitement de troubles gastro-intestinaux, **caractérisée en ce que** le composé inhibiteur de la pompe à protons est choisi dans le groupe des composés connus sous les noms génériques oméprazole, lansoprazole, pantoprazole, rabéprazole, ténatoprazole et ésoméprazole ou un sel pharmaceutiquement acceptable de ceux-ci et les pastilles sont dispersées dans un milieu aqueux visqueux pharmaceutiquement acceptable choisi parmi le yaourt, le lait fermenté, un sirop et les liquides aqueux ayant une viscosité analogue, pour l'administration par un tube gastrique à des patients pédiatriques ayant besoin d'un tel traitement.

5. Utilisation selon la revendication 4,
**caractérisée en ce que** le milieu aqueux visqueux est un sirop de sucre ayant une teneur en sucre d'au moins 63 % en poids.

6. Utilisation selon l'une ou l'autre des revendications 1 et 4, **caractérisée en ce que** la viscosité de la formulation après gélation doit être de 0,005-10 Pa s telle que déterminée à un taux de cisaillement de 10 s⁻¹ à partir d'une courbe d'écoulement enregistrée sur un rhéomètre muni d'une géométrie plan-plan.

7. Utilisation selon l'une ou l'autre des revendications 1 et 4, **caractérisée en ce que** la viscosité de la formulation après gélation doit être de 0,05-5 Pa s telle que déterminée à un taux de cisaillement de 10 s⁻¹ à partir d'une courbe d'écoulement enregistrée sur un rhéomètre muni d'une géométrie plan-plan.

8. Utilisation selon l'une ou l'autre des revendications 1 et 4, **caractérisée en ce que** la suspension aqueuse est administrée par des tubes ayant la taille CH 5 à CH 10 (CH = Cherrier).

9. Utilisation selon l'une quelconque des revendications 1 à 8, **caractérisée en ce que** la quantité de la dose de la substance active administrée est de 0,5 - 40 mg.

10. Utilisation selon la revendication 1,
**caractérisée en ce que** le support aqueux est choisi dans le groupe constitué d'eau, de jus de fruit, de sirop et de produits laitiers.

11. Utilisation selon l'une quelconque des revendications 1 à 8, **caractérisée en ce que** la quantité de milieu visqueux administré est d'environ 1 - 35 ml.

12. Formulation pharmaceutique destinée à l'administration par un tube gastrique à des patients pédiatriques comprenant un inhibiteur de la pompe à protons sous la forme de pastilles revêtues d'enrobage entérique, **caractérisée en ce que** le composé inhibiteur de la pompe à protons est choisi dans le groupe des composés connus sous les noms génériques oméprazole, lansoprazole, pantoprazole, rabéprazole, ténatoprazole et ésoméprazole ou un sel pharmaceutiquement acceptable de ceux-ci et les pastilles sont en mélange avec un ou plusieurs épaississants pharmaceutiquement acceptables choisis dans le groupe comprenant l'amidon, la gomme xanthane, le carraghénane, la gomme guar, la gomme de caroube, la tragacanthe, la gélatine, la pectine et les dérivés de cellulose modifiée dispersés dans un support aqueux.

13. Utilisation selon l'une quelconque des revendications 1 à 8, **caractérisée en ce que** les pastilles à enrobage entérique sont sphériques et ont une taille inférieure à 1 mm.

14. Utilisation selon l'une quelconque des revendications 1 à 8, **caractérisée en ce que** les pastilles à enrobage entérique sont sphériques et ont une taille inférieure à 0,5 mm.

15. Utilisation d'un épaississant dans la préparation d'une composition aqueuse visqueuse destinée à l'administration par un tube gastrique pour le traitement de troubles gastro-intestinaux, **caractérisée en ce que** la composition comprend un inhibiteur de la pompe à protons choisi dans le groupe des composés connus sous les noms génériques oméprazole, lansoprazole, pantoprazole, rabéprazole, ténatoprazole et ésoméprazole ou un sel pharmaceutiquement acceptable de ceux-ci et sous la forme d'un multiple de pastilles revêtues d'enrobage entérique en mélange avec un ou plusieurs épaississants choisis dans le groupe comprenant l'amidon, la gomme xanthane, le carraghénane, la gomme guar, la gomme de caroube, la tragacanthe, la gélatine, la pectine et les dérivés de cellulose modifiée, et un support aqueux.

16. Utilisation d'un milieu visqueux dans la préparation d'une composition aqueuse visqueuse destinée à l'administration par un tube gastrique pour le traitement de troubles gastro-intestinaux, **caractérisée en ce que** la composition comprend un inhibiteur de la pompe à protons choisi dans le groupe des composés connus sous les noms génériques oméprazole, lansoprazole, pantoprazole, rabéprazole, ténatoprazole et ésoméprazole ou un sel pharmaceutiquement acceptable de ceux-ci et sous la forme d'un multiple de pastilles revêtues d'enrobage entérique en mélange avec un milieu aqueux visqueux choisi parmi le yaourt, le lait fermenté, un sirop et les liquides aqueux ayant une viscosité analogue.
